# EUROPEAN PATENT APPLICATION

(11) **EP 1 447 454 A1**
(43) Date of publication of application: **18.08.2004**
(21) Application number: 03003407.8
(22) Date of filing: 14.02.2003
(51) Int. Cl.: C12Q 1/68, G01N 21/62, G01N 35/02, G06F 19/00, B01J 19/00

(54) **Method and apparatus for detecting pathogens**

(71) Applicant: DR. Chip Biotechnology Incorporation, Junan Township, Miaoli County, Taiwan 350 (TW)
(72) Inventor: Lin, Chia-Po No. 31, Ke Jung Rd., Chu-Nan 350, Miao-Li County (TW); Chen, Chien-An No. 31, Ke Jung Rd., Chu-Nan 350, Miao-Li County (TW); Chen, Meng-Yu No.31, Ke Jung Rd., Chu-Nan 350, Miao-Li County (TW); Huang, Ming-Yuan No. 31, Ke Jung Rd., Chu-Nan 350, Miao-Li County (TW)
(74) Representative: Becker Kurig Straus

(57) **Abstract**

The present invention describes a system and a method for detecting the presence of a pathogen in a product material, such as food, cosmetics or pharmaceuticals. The system comprises a mobile tray for carrying a micro-array substrate, a light source, a series of mirrors, a lens and a detector for capturing the image of the micro-array. The mobile tray is adapted to carry micro-arrays of different sizes, such as a slide or a microplate. To obtain a high quality micro-array image, a series of mirrors and lenses are used for manipulating the light path and focus. A software with pattern recognition is included for analysis and matching the result of biochemical reaction.

SEQUENCE LISTING

<110>  DR. Chip Biotechnology Incorporation

<120>  Micro-array image detection and recognition apparatus

<130>  51362 EP

<140>  EP 03 003 407.8
<141>  2003-02-14

<160>  4

<170>  PatentIn version 3.1

<210>  1
<211>  24
<212>  DNA
<213>  Artificial Sequence

<220>
<223>  primer

<400>  1
tgaatgcgca agctgaaaaa gtag                                              24


<210>  2
<211>  24
<212>  DNA
<213>  Artificial Sequence

<220>
<223>  primer

<400>  2
acgccgttag gtgtattgat tgtg                                              24


<210>  3
<211>  26
<212>  DNA
<213>  Artificial Sequence

<220>
<223>  primer

<400>  3
atggttttgg tagaattggt cgttta                                            26


<210>  4
<211>  25
<212>  DNA
<213>  Artificial Sequence

<220>
<223>  primer

<400>  4
gacatttcgt tatcatacca agctg                                             25

## Description

The present invention pertains to an assay and a system for easily and rapidly detecting the presence of a variety of pathogens in a sample. In particular, the present invention relates to the field of image capture and pattern recognition in a micro-array, specifically in combination with molecular biological analysis methods and equipment to allow an automated an rapid operation.

Due to an increasing and repeated incidence of microbial contamination of material, such as food, cosmetics or pharmaceuticals, and its sometimes pernicious effect on human health, the microbial safety thereof has become a major concern for producers and consumers alike. In general, these materials may be contaminated by a variety of different microbes, examples of which are *Listeria* or enteric bacteria, such as *Shigella, Salmonella,* or strains of *Escherichia coli*, all of which are known to be a major source of diarrhea and enteric fever in man leading to more than about three million deaths per year.

Among the number of pathogens found *Salmonella,* in particular *S*. *typhimurium* is one of the most common agents poisoning in particular food material. Since the intestinal tract is its natural habitat, *Salmonella* enters the human food chain mainly through foods of animal origin, including poultry, beef, pork, cheese, mayonnaise, baby formulas and canned sea food.

Another foodborne pathogen is represented by *Escherichia coli*, e.g. Enterohemorrhagic *E. coli* (EHEC), which is transmitted largely through ingestion of contaminated food, distinctively undercooked meat. By and large, EHEC infestation has been attributed to the presence of the micro-organisms in ground beef, since beef and dairy cattle harbour the pathogen in their intestinal tracts, with carcasses frequently getting contaminated during the slaughter process. In humans, infestation with EHEC leads to several severe clinical conditions, including hemorrhagic colitis (HC) or the hemolytic uremic syndrome (HUS), during which cells lining blood vessels are destroyed by toxins secreted by the pathogen, which may eventually result in a permanent kidney damage or even kidney failure. Although EHEC is acknowledged to cause serious illness even in healthy adults, infants or young children, elderly and immune-compromised individuals are at a particular risk of even dying or suffering permanent damage from such an infection.

Another prominent example of a pathogen is *Listeria monocytogenes*, a motile pathogen which may cause a number of diseases in humans, including meningoencephalitis, infectious mononucleosis-like syndrome, pneumonia and urethritis. It is known that the pathogen may be transmitted from the consumption of infected meat or milk.

In order to prevent distribution of contaminated substances to consumers, the producers regularly subject the respective material to assays for determining the presence or absence of pathogens therein.

So far several methods are applied, most of which are based on long standing traditional principles. These principles comprise enriching the pathogen by culturing the sample for several days in a nutrient, which may optionally be selected such that it promotes the growth of the target micro-organism, while suppressing the growth of other micro-organisms. The target micro-organism is subsequently isolated and purified, followed by a determination of the physiological and biochemical characteristics of the micro-organisms and/or the antigenic profile of the culture.

These conventional methods do, however, suffer from a variety of significant problems. The procedure requires a number of days before results may be obtained, which time period is in most cases too long, since the materials under investigation should be released without delay for use in production and/or distribution. In addition, the growth of target microorganisms in the enrichment media is complicated, requires a number of different chemicals and is sometimes unpredictable, since the presence of fast-growing non-target micro-organisms may interfere with the growth of the target micro-organisms.

With the progress of antibody techniques a number of alternative procedures have been elaborated, including fluorescent antibody tests, enzyme linked immunosorbent assay ("ELISA"). However, these techniques require a high number of target cells to ensure the presence of an adequate concentration of the target micro-organisms in the enrichment broth so as to reliably detect it.

In order to increase specificity and to reduce the number of detectable microbial cells, gene technology techniques are put to use, which are based on nucleic acid-amplification technologies (PCR) and eventually hybridization reactions with pathogen-specific gene probes. Here, numerous options exist for detecting hybridization reactions, e.g. radioactivity, chemi-luminescence, confocal laser-induced fluorescence, colorimetry, electrochemistry, and surface resonance etc.. Radioactivity is sensitive, but hazardous to the environment and the user, so that its applicability is limited. It requires an X-ray film development to reveal the results, which is single color detection and adds to the time required for the assessment. Another popular technique for detecting hybridization results is confocal laser-induced fluorescence. The fluorescence dye in two or more colors is incorporated in targets or subsequent reaction products. The fluorescence intensity of different wavelength of each dot is compared to its local background around the dot. The detector of fluorescence converts the low levels of light to an electrical signal. The detectors may be photo multiplier tubes (PMTs), avanlanche photodiodes (APDs) and CCD arrays. Yet, the limitation of confocal laser systems is the reading area and the reading speed. In addition, the cost of a laser-induced fluorescence detection systems and fluorescence labels can be prohibitively high for many users. An alternative to fluorescence detections are enzyme colorimetric detections. The enzyme colorimetric method uses an enzyme linked to a target DNA, which will hybridize with specific immobilized probes on a carrier. Color will develop as enzymes react with color substrate such as alkaline phosphatase, horseradish peroxidase etc..

So far the existing procedures are still too time consuming and specifically still lack the required specificity to identify a pathogen also at a sub-genus level. At present, there is no rapid and reliable assay and system available for detecting microbial pathogens at early stages of contamination of a given material.

Thus, a problem of the present invention resides in providing a highly sensitive and specific process and system which can be used for a rapid detection of target microorganisms in a sample.

The above problem has been solved by providing an assay for determining the presence or absence of a pathogen in a product sample, which comprises the steps of (a) subjecting a sample to be tested to conditions, wherein nucleic acids are released from cells, (b) specifically amplifying a part of at least one target nucleic acid derived from a pathogen by using primers, specific for the target nucleic acid, (c) transferring the amplified nucleic acid to a carrier, which contains on pre-selected locations thereof a nucleic acid sequence complementary to at least a part of the target nucleic acid amplified in step (b) under conditions, allowing hybridization of complementary strands, and (d) detecting a potential hybridization event at any of the locations on the carrier, wherein the pattern of detected hybridization signals is indicative of the presence or absence of a given pathogen.

In the figures,
Fig. 1 shows sample pattern images obtained when carrying out the assay of the present invention;
Fig. 2 shows two examples of carriers to be used according to the present invention;
Figs. 3 A and B show a schematic array of an arrangement of a light source and mirrors in the apparatus according to the present invention;
Fig. 4 depicts a part of the apparatus of the present invention including the moveable tray;

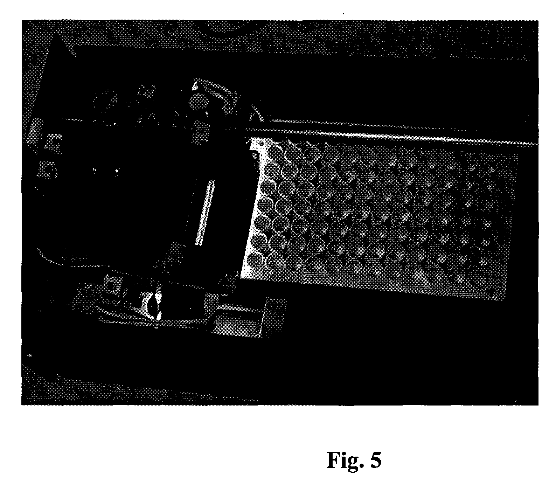

Fig. 5 shows a 96 well microtiter plate as a carrier in a tray; and

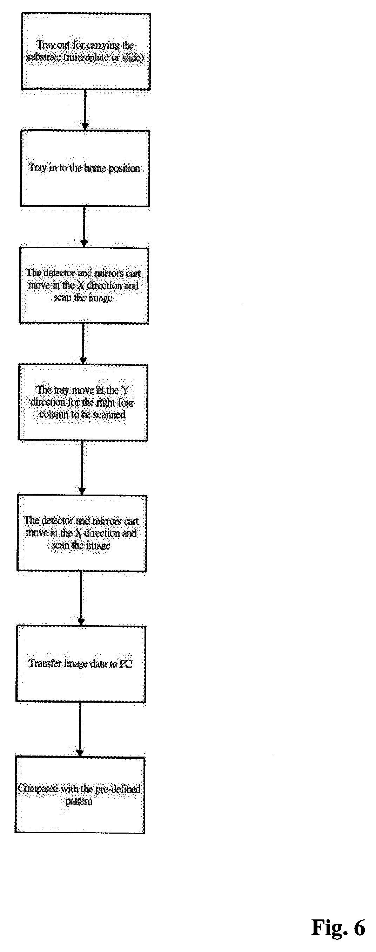

Fig. 6 shows a flow chart for moving the tray in the apparatus.
Fig 7 is a view on a microplate with square wells; and
Fig. 8 shows the part of the apparatus shown in Fig. 4 in a casing.

Generally speaking, the assay of the invention is designed to quickly and reliably determine the presence of target pathogens in a product sample, e.g in solid or liquid food material, such as meat or milk, or in cosmetics or pharmaceuticals etc.. In particular, due to including several basic "exclusion" steps in the assay, the present process rapidly provides results with a high reliability and specificity. The first basic exclusion step may be seen in using primers specific for a given pathogen, which primers should in general allow formation of target nucleic acids only, that will exhibit a known length and a known nucleic acid sequence. It will of course be understood, that in case a higher specificity is desired, nested PCR may be envisaged, which decision will be made by the practitioner based on his own skills and knowledge on site. The next basic exclusion step in the present assay may be seen in selecting specific probes, which are immobilized on the carrier. The probes are chosen such that they are complementary to at least a part of the nucleic acid stretch amplified before, preferably without overlapping with primer sequences. Consequently, even in case that in the amplifying step, a stretch of nucleic acid has been synthesized, which is not specific for a/the given pathogen, this stretch of nucleic acid will not hybridize with the probe immobilized on the carrier. Apart from selecting appropriate hybridization conditions to allow base hybridization of complementary strands only, this second basic exclusion step may be supported by selecting washing conditions of high stringency. In consequence, false results may be avoided without impairing the speed with which the assay may be performed.

The assay includes in a first step raw preparation of nucleic acids from cells present in the sample.

Optionally, before isolating nucleic acids from the sample, it may be transferred to a nutrient, such as LB-broth, supporting the growth of the pathogens to be assayed. The sample may be incubated in the nutrient for about 8 - 16 hours, e.g. over night, so as to increase the number of pathogen cells therein. The medium may contain an agent, such as esculin, which will change the color of the medium in the presence of the target pathogen. If such a characteristic color change is observed, the motility characteristics of the pathogen for the color change may be determined as well. In addition, the medium may also contain agents promoting the growth of the target pathogen. In particularly preferred forms, the growth medium is Fraser Broth, a known medium commercialized by, inter alia, Becton Dickinson Microbiology Systems of Cockeysville, Md. While Fraser Broth is a preferred medium, those skilled in the art will appreciate that other media can also be employed, so long as the media contains the described color-change agent.

Cells contained in the sample or optionally cells grown in the nutrient may be isolated by conventional means, such as centrifugation, e.g. at about 6000 g for 5 minutes. In case of a solid sample, such as e.g. meat, the sample may optionally be incubated in a medium containing trypsin, so as to disintegrate the solid mass and release any pathogen present in the tissue.

The cells thus obtained are then treated in a conventional way to release nucleic acids present therein, such as e.g. a lysis buffer and/or application of heat. According to a preferred embodiment the lysis buffer may be designed such that it preferentially lyses bacterial cells.

In case ribonucleic acid (RNA) is the target nucleic acid, the lysis buffer and preferably all subsequent buffers may contain means to prevent degradation of the RNA, such as e.g. DEPC, which blocks activity of RNAse. In case DNA is the target nucleic acid, use of means preventing degradation thereof may be omitted, since DNA is in general more stable. The solution containing the nucleic acid is ordinarily stored at 4 °C and may be used immediately or stored in case of need under appropriate conditions.

In a next step (step (b)) the nucleic acid isolated, or an aliquot thereof, is subjected to an amplification step via PCR. Methods of carrying out a PCR are well known in the art and all, buffers, enzymes, reagents are commercially available, together with the manufacturer's instructions. As primers for the PCR reaction nucleic acids specifically binding to individual nucleic acids selectively present only in the target pathogens to be assayed are utilized.

Examples for such primers are given below:

### Escherichia coli:

In general *E. coli*-specific primers may be derived from nucleotide sequence between 81889 and 83238 of *E. coli* genome (GenBank Accession No. AP002562). Preferably primers may be represented by a 24 oligonucleotide N1 (5'-TGAATGCGCAAGCTGAAAAAGTAG-3'; corresponding to nucleotides 82568-82591 of GenBank Accession No. AP002562), and an reverse primer being a 24 oligonucleotide N2 (5'-ACGCCGTTAGGT GTATTGATTGTG-3', corresponding to a complementary sequence of nucleotides 83075-83052 of GenBank Accession No. AP002562). Alternatively, the primer pair to be utilized is, the forward primer is the 18 oligonucleotide located at the 3'-end of N1, and likewise, the reverse primer is a 8 oligonucleotide located at the 3'-end of N2.

### Staphylococcus aureus:

Primer for specifically amplifying DNA present in *Staphylococcus aureus* are e.g. 5'-ATG GTTTTGGTAGAATTGGTCGTTTA-3' and 5'-GACATTTCGTTATCATACCAAGCTG-3', which amplify part of the gap region in *Staphylococcus* species, by which a variety of different *Staphylococcus* species may be distinguished as well.

As an exemplary protocol for carrying out a PCR the following scheme may be noted : 94 °C - 5 min; 30 cycles : 94 °C - 40 sec, 55 °C - 40 sec, 72 °C- 40 sec; then 72 °C - 6 min. Accoring to a preferred embodiment the primers used in the PCR are biotinylated at their 5'-end, so as to allow an easy and rapid detection of the amplified material at a later stage. The amplified material may be used immediately in the hybridization step or stored at -20 °C.

In step (c) the material amplified, or an aliquot thereof, is hybridized to a probe, immobilized on a carrier. Methods, conditions and buffers for immobilizing nucleic acids on a carrier and for performing hybridization are well known in the art. Prior to the hybridization reaction the amplified material is denatured by e.g. heating in boiling water for 5 minutes, and transferred to the hybridization reaction. It will be acknowledged by the skilled person that the conditions, in particular the buffer ingredients and the concentration thereof, as well as the temperature at which the hybridization is carried out may be selected by the skilled person considering the length and the base composition of the expected hybridization region such that only complementary strands are allowed to bind. In general hybridization may be carried out at a temperature of from 45 °C to 65 °C for about ½ to about 3 hours.

After completion of the hybridization the carrier may be subjected to one or more washing steps, with the washing buffer (salt and concentration thereof) and the temperature of the washing process being selected such that only complementary strands are allowed to stay bound to the probes immobilized on the carrier, while non-specifically bound nucleic acids are washed away.

After the optional washing step, during step (d), bound nucleic acids are detected. The skilled person is well aware of a number of different ways to detect nucleic acids bound to a probe. According to a preferred embodiment the bound nucleic acid, that contains a primer used in the amplification reaction, is linked to biotin, against which a number of antibodies (conjugates) are commercially available. The antibody binds to biotin. In a next step a second antibody directed to the first antibody and linked to a marker may be applied. The marker may be selected according to the specific needs, but is preferably a dye, a fluorescent agent or an enzyme. Alternatively, the marker may also be linked to the first antibody. According to a preferred embodiment the carrier is contacted with a Streptavidin - Alkaline Phospatase (Strept-AP) to bind to biotin present on the nucleic acid hybridized to the respective probe. Subsequently, a substrate for the alkaline phosphatase is provided, which gives a color as a result of the enzymatic activity.

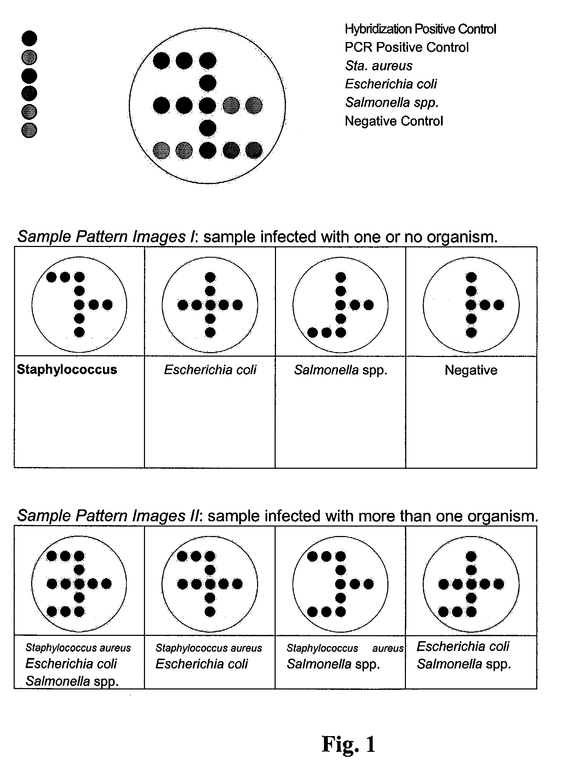

The result of the above assay is now interpreted by the specific pattern formed on the carrier. A pattern specific for a given pathogen may be achieved by designing the carrier such that at specific locations thereon oligonucleotides probes are pre-spotted to capture specific nucleic acid targets during the hybridization reaction. Besides hybridization positive control and PCR positive control, only the specific pathogens present in the sample will hybridize with target probes and result, via the formation of detectable marker on the carrier after the hybridization reaction and marker detection, in the formation of a pattern, specific for a given pathogen. The hybridization positive control and PCR positive control shall always be observed once the reaction is completed. Missing any of these dots suggest an operation error. A preferred embodiment is shown in Fig. 1, which illusrtates a well of a 96 well micro-plate and the pattern for different micro-organisms. It will be acknowledged that any kind of pattern may be envisaged, with the proviso that the software will recognize and attribute the pattern to the particular micro-organism. The pattern may be obtained by pre-spotting the same probe at the locations selected or by pre-spotting different probes, all selective for one micro-organism, at the locations selected, which latter array will additionally increase the specificity of the assay.

As may be seen the present assay allows a determination of the presence or absence of a number of different micro-organisms in both viable and non-viable form on one carrier at the same time. The combination of "exclusion" means together with the design of the carrier enables a rapid and highy reliable assay, with which a number of different pathogens may be assessed in a sample. Also, since the carrier may be designed to harbor a high number of carriers, such as e.g. a micro-plate with 96 wells, on the bottom of which such carriers are arranged, a total of 96 different samples may be processed at the same time adding to the time saving advantages obtainable according to the present invention.

According to another aspect the present invention relates to a system for performing an assay for determining the presence or absence of a pathogen in a product material, which has a high throughput ability, is compatible with existing medical or biological standard, and represents a cost effective micro-array system for regular uses.

The present system/apparatus comprises as basic components, a bioreader, containing a movable tray to carry and to move a micro-array, at least one light source, a focus lens, a series of mirrors to adjust the light path, and a line CCD.

Automation, throughput and cost effectiveness are the major considerations of a micro-array for regular uses (for example clinical diagnostics). In the present invention, a microplate is shown, in which the probes (DNA or protein) are pre-deposited on the bottom of the wells in the microplate to make a micro-array. In this exemplary case, every well of the microplate represents a single micro-array to make a total of 96 micro-arrays per microplate. This allows that the microplate-based micro-array is compatible with existing automated systems of most laboratories. The parallel analysis function of a micro-array also meets the requirement of high throughput and cost effectiveness for regular uses.

The advantage of the enzyme colorimetric method disclosed herein is its cost effectiveness, which fits the needs of regular uses and high throughput for diagnostic applications. The system of the present invention is compatible with micro-plates, which is the standard for medical, biological and diagnostic applications.

However, in general the use of micro-plates creates some problems.

First, the depth and diameter ratio of wells in a microplate is about 1.6 or more, which renders it difficult to project light onto the array located on the botton of the wells. In contrast thereto, micro-arrays of the prior art, which are spotted on a flat surface with no sidewalls do not cause any problems in this respect and the light can be projected from essentially any direction.

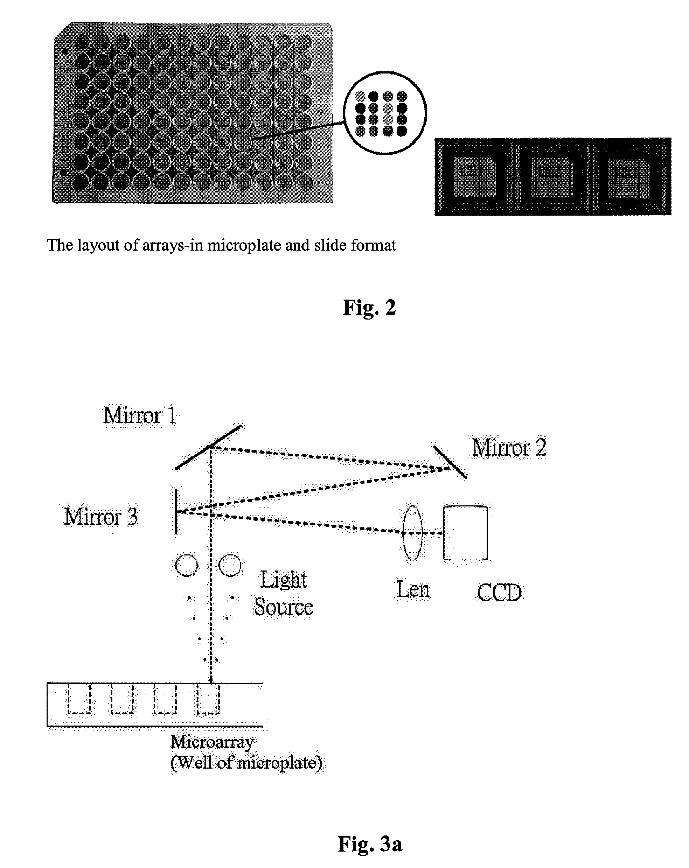

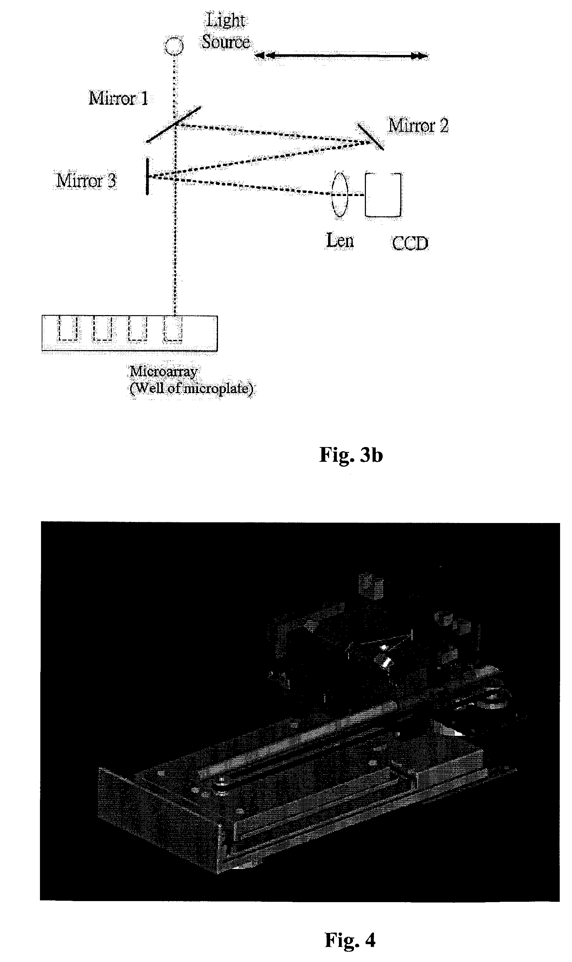

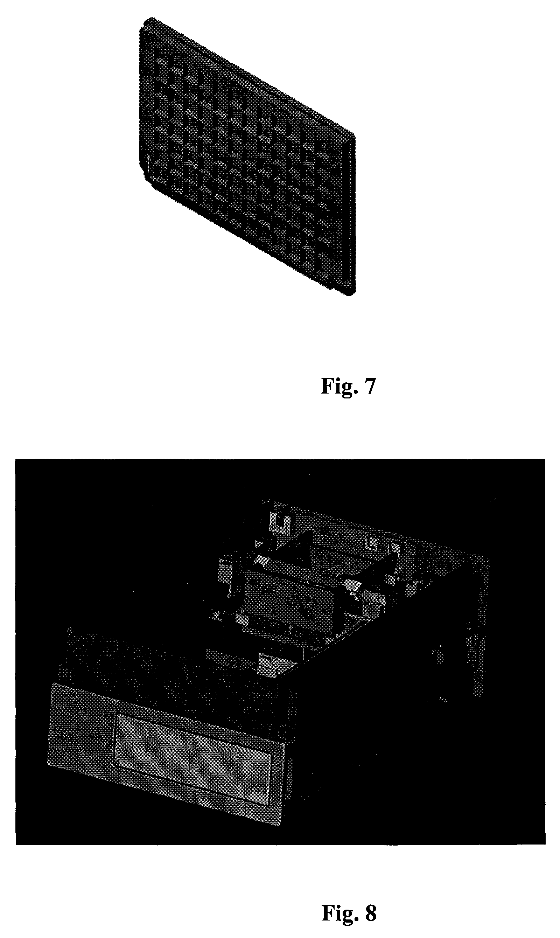

In order to obtain a good image of the arrays in the microplate though, the light must be evenly distributed in the well without interfering with the surrounding wall(s). According to the present invention this may be achieved by arranging the at least one light source in a specific manner and optionally applying supporting means, or using a modified micro-plate. First, two linear light sources may be used under a mirror 3 (cf. Fig. 3). Second, the light source may be positioned upright to the well and light is passed through a dichroic mirror (as a supporting means) to project into the wells. Third, the microplate may be modified as is shown in Fig. 7. Such a new type of microplate is designed with square wells to allow light to project into it easier. The squared-wells are also convenient for micro-array spotting and for reducing the reaction volume of the reagents. According to a preferred embodiment the light source projects light into wells in a microplate just upright as is shown in Fig. 3.

The second difficulty is the large reading area of the microplate, that causes an inherent distortion problem when acquiring the image.

In order to minimize the problem of image distortion, the present system/apparatus comprises mirrors to extend the light path from the objects to the detector. In addition, a second means to solve such problem is to utilize a focus lens. In case, as is exemplarily shown in Fig. 3, a light source is on the upright to the mirror 1 (dichroic mirror), light passes through the mirror 1 to project onto the well substrate. The reflecting light from the well substrate will be projected on the mirrors 1, 2, 3, then passes through the focus lens to a CCD. The CCD is a line arrays type. Therefore, the mirrors 1,2,3, the focus lens and the CCD are compacted to a cart, which can move in the X- and/or Y-direction. Additionally, in order to reduce the deep well distortion, the substrate may be divided in two parts. In addition, in order to further improve the image, a double scan technique may be applied. The whole microplate is scanned twice with four columns at a time.

To reduce variation of taking the image, all of the mirror(s), lens and detector are preferably arranged on a moveable cart. If the cart has a Y-direction mobility, the light path can then be constrained (remain the same light path) from the first well to the last (since the light source may be moved in the X-direction, if desired, while keeping the same distance to the micro-plate).

Another problem resides in taking the image as such. Normally, to gain each image of the wells in a microplate, a CCD camera is to picture each single well. Proceeding accordingly would normally take ninety-six efforts to get a complete reading of a microplate, which takes a long time, causes a big amount of data to process and limits the transferring speed. In the present system/apparatus, a double line-scan technique may be applied, with the advantage of reducing the data size and the image capturing time. A processor may be used to control the motion of the reader, to acquire the image data and to transfer data to computer for micro-array image pattern recognition.

The result of the micro-array assessment is detected by fluorescent or colorimetric substrate labels. The optical sensors are devices to capture the light emitted from the fluorescent substrates or the color developed from colorimetric substrates. A micro-array reader is a device, which captures the light reflected or emitted from the micro-array. Usually, the biochip readers are designed for slide format (1 inch x 3 inch).

According to a preferred embodiment the carrier is a microplate, that may carry 96 tests in one plate (or more, depending on the number of wells in the plate) and there are plenty of machines available to automate the microplate operation. Also, in this invention, instead of visual determination a reader is applied to obtain the image and a software, to analyze the result. This not only give a qualitative but also a quantitative reading with an automatic data analysis and output. With these two major break throughs, the present system apparatus is much closer to regular uses required in the laboratory.

According to another embodiment the present invention pertains to an automated method for determining the presence of a pathogen in a product sample which comprises the steps of (i) transferring the carrier, on which an assay for determining the presence of a pathogen in a product sample has been carried out, on a tray contained in the present system, (ii) moving the tray in the home position, (iii) moving the detector and mirror carts in the either of X- or Y-direction and scan the image; (iv) moving the tray in the other of Y- or X-direction for columns to be scanned; (v) if desired, moving the detector and mirror carts in the either of X- or Y-direction and scan additional images; (vi) transfer images to a computer; and (vii) comparing the pattern obtained with a predefined pattern.

Most of the prior art software is designed to monitor different gene expression profiles. The function is to search the dots and to compare the intensity of different color spectrums of the same dot. In the present invention, the software is designed primarily to identify features (for example pathogens) of testing samples. The main function of the software is to recognize patterns in the micro-array. The standard patterns are predefined and may also be defined by the user. The software may locate the arrays position and the dots above a set threshold. After pattern reorganization, the results then can be analyzed with built-in analysis tool and can be output as in spreadsheet format.

Therefore, according to a further embodiment the present invention also pertains to a software tool or a computer program product comprising program code means stored on a computer readable medium for carrying out the above automated method for determining the presence of a pathogen in a product sample. The computer program product may comprise program codes, downloadable from a server for carrying out the above method. In addition also a computer data signal embodied in a carrier wave and representing a program that instructs a computer to perform the steps of the above method is envisaged by the present invention.

The following examples illustrate the invention without limiting it thereto.

### Examples

### I. Materials

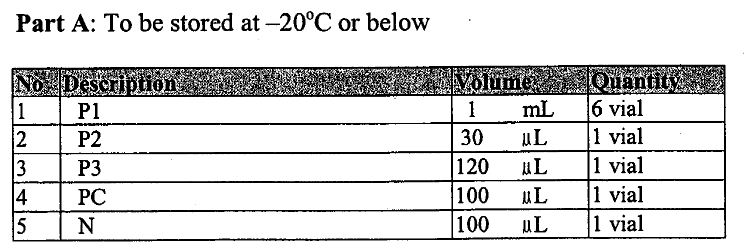

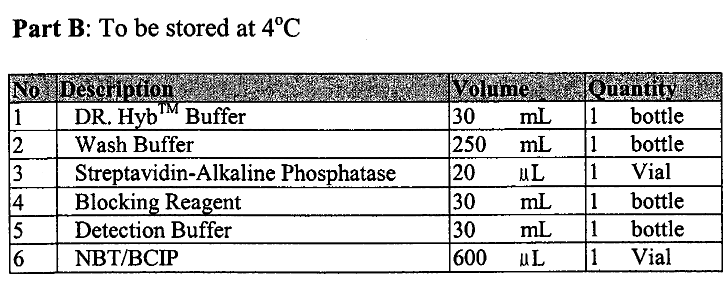

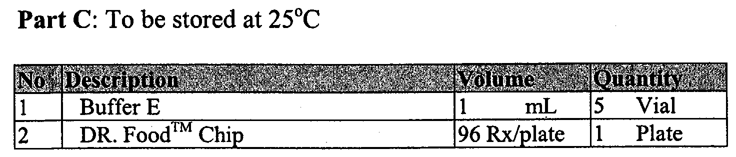

| PRODUCT DESCRIPTIONS | |
|---|---|
| **Buffer E** | Lysis buffer (cf. Maniatis, A Laboratory Manual, Cold Spring Harbor, 1992) |
| **P1** | PCR premix including dNTPs, PCR buffer, magnesium, and specific 5'-biotin-labeled primer pairs for amplification of *Staphylococcus aureus*, *Escherichia coli*, *Salmonella* spp. and internal control (P3); (according to the manufacturers instructions; cf. also Maniatis, supra) |
| **P2** | Taq DNA polymerase (5U/µL) (Boehringer Mannheim). |
| **P3** | Internal control DNA for monitoring each PCR reaction. |
| **PC** | Positive control DNA template |
| **N** | Negative control |
| **DR. Hyb™ Buffer** | Hybridization buffer (cf Maniatis, supra) |
| **Wash Buffer** | For stringency wash after hybridization reaction (cf. Maniatis, supra). |
| **Streptavidin-Alkaline Phosphatase** (Strept-AP) | A secondary antibody conjugates (AP) for the detection of biotinylated molecules; The recommended working dilution for the labeled antibodies is 1:2000 |
| **Blocking Reagent** | Reduce non-specific nucleic acid binding to polymer chip surfaces. Also as a diluent for Strept-AP (cf. Maniatis, supra) |
| **Detection Buffer** | A diluent for NBT/BCIP to ensure the activity of alkaline phosphatase. (cf. Maniatis, supra) |
| **NBT/BCIP** | The solution is used for detection of alkaline phosphatase. BCIP is the AP-substrate which reacts further after the dephosphorylation to give a dark-blue indigo-dye as an oxidation product. NBT serves as the oxidant and gives also a dark-blue dye. |
| **DR. Food™ Chip** | A platform for hybridization reaction. Its surface is pre-spotted with a variety of specific probes for target organisms. |

### II. Sample

As a sample to be tested 100 ground beef has been used, to which 100 cfu of the respective micro-organisms have been added and mixed therein. 2 g of the ground beef has been suspended in 5 ml of PBS by vigorously vortexing for 2 minutes. The suspension was centrifuged at low speed (200 g / 4 °C) for 2 minutes to remove heavy and non suspended matter. 1 ml of the suspension has been transferred to 10 ml LB broth and incubated over night at 37°C (96 % humidity).

### III. DNA Extraction

1 ml of the enrichment culture and 1 ml of the original sample are transferred to a microtube and centrifuged at 6,000 g for 5 minutes. The supernatant was removed and the pellet was resuspended in 50 µL Buffer E and vortexed until completely mixed. The solution was heated in boiling water for 10 minutes, chilled on ice for 2 minutes and centrifuged at 6000 g for 5 min. The supernatant was collected transferred to a new tube and put on ice until further use.

### IV. DNA Amplification

For the amplification process a PCR master mix has been prepared by pipetting the following reagents into a PCR tube and mixing. For each batch of the test additional tubes were prepared as negative and positive control.

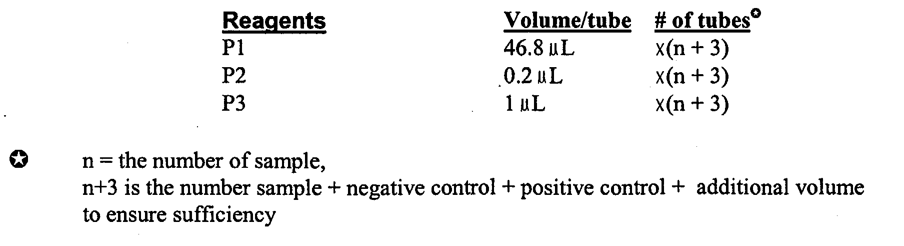

48 µl of the master mix were pipetted into each PCR tube. 2 µl of each of the isolated DNA were transferred into each tube and mixed by pipetting. For the PCR negative control 2 µl N (Negative control) and for the PCR positive control 2 µl PC (positive control template) were utilized. The final volume for each tube was 50 µl.

The tubes were placed in a Thermo cycler (Perkin Elmer) and the amplification program listed below was run

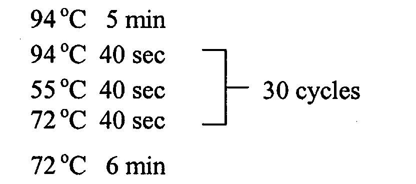

After completion the tubes were put on ice.

### V. Hybridization

200 ml of DR. Hyb Buffer were pipetted in a reaction well and pre-heated to 50 °C in an oven. 10 µl of the amplified material were transferred into a tube and mixed with 190 µl of DR. Hyb Buffer. The tube was heated in boiling water for 5 minutes to achieve denaturation and subsequently chilled on ice. The hybridization mixture was then transferred into the reaction well and incubated at 50 °C for 1 hour.

### VI. Stringency Wash

After hybridization the hybridization buffer was removed from the wells and the wells were rinsed with 200 µl Wash buffer. The washing step was repeated twice.

### VII. Detection

0.1 µl Strept-AP was mixed with 200 µl Blocking Reagent (1:2000) and the mixture was transferred into the well, allowing reaction for 30 minutes at room temperature. Subsequently the mixture was poured off and the wells were rinsed twice with 200 µl Wash Buffer, twice. 4 µl NBT/BCIP were mixed 196 µL Detection Buffer for each reaction, and the mixture was transferred into each well. The plate was placed in a dark condition (chamber/room) for 10 minutes at room temperature. The wells were rinsed twice with deionized water and dried before reading the image.

For reading the plate was placed in the apparatus of the present invention (DR. AIM™ Reader) to read the result pattern in the well. The pattern as shown in Fig 1 was obtained.

## Claims

1. An assay for determining the presence or absence of a pathogen in a sample which comprises the steps of
(a) subjecting a sample to be tested to conditions, wherein nucleic acids are released from cells,
(b) specifically amplifying at least a part of at least one target nucleic acid by using primers, specific for one or more selected pathogen(s),
(c) transferring the amplified nucleic acid to a carrier, which contains on preselected locations thereof a nucleic acid sequence complementary to at least a part of the target nucleic acid amplified in step (b) under conditions, allowing hybridization of complementary strands, and
(d) detecting a potential hybridization event at any of the locations on the carrier, wherein the pattern of detected hybridization signals is indicative of the presence or absence of a given pathogen.

2. The assay according to claim 1, wherein an enrichment step is carried out prior to step (a).

3. The assay according to claim 1 or 2, wherein the pathogen is selected from the genus *Staphylococcus, Escherichia coli* and/or *Salmonella.*

4. The assay according to any of the preceding claims, wherein the detection of the hybridization signal is effected by means of color development.

5. The assay according to claim 4, wherein in step (b) primers are utilized, that are linked to biotin at their 5'-end and wherein in step (d) biotin-specific antibodies are applied.

6. The assay according to any of the preceding claims, wherein the carrier contains DNA sequences at pre-selected locations of at least two of the target micro-organisms.

7. A system/apparatus for carrying out an assay according to any of the preceding claims which comprises
(i) a carrier, which contains on pre-selected locations a DNA-sequence specific for a given pathogen and forms a micro-array;
(ii) a bioreader, comprising a movable tray to carry the micro-array in X- and/or Y-directions;
(iii) at least one light source including a focus lens, and
(iv) an analysis software.

8. The system according to claim 7, wherein the carrier is a 96 well micro-plate.

9. The system according to claim 7 or 8, wherein at least two light sources are used.

10. The system according to claim 9 , wherein a series of mirrors are included to extend the light path.

11. The system according to any of the preceding claims, wherein the light source is positioned upright to the well and the light is passed through a dichroic mirror to project into the well.

12. The system according to any of the claims 7 to 11, wherein the micro-array is scanned two lines at least at the same time.

13. The system according to any of the claims 7 to 12, wherein the software is designed to recognize patterns and output it on a screen.

14. An automated method for determining the presence of a pathogen in a product sample which comprises the steps of
(i) transferring the carrier, on which an assay for determining the presence of a pathogen in a product sample has been carried out, on the tray contained in the system of claim 7,
(ii) moving the tray in the home position,
(iii) moving the detector and mirror carts in either of X- or Y-direction and scan the image,
(iv) moving the tray in the other of Y- or X-direction for columns to be scanned;
(v) if desired moving the detector and mirror carts in the either of X- or Y-direction and scan additional images;
(vi) transferring images to a computer; and
(vii) comparing the pattern obtained with a predefined pattern.

15. The method according to claim 14, wherein the carrier is a microtiter-plate, preferably a 96 microtiter-plate, preferably a 96 microtiter-plate with square wells.

16. Software tool comprising program code means stored on a computer readable medium for carrying out the method according to claim 14 or 15.

17. Computer program product comprising program code means stored on a computer readable medium for carrying out the method according to claim 14 or 15.

18. Computer program product comprising program code, downloadable from a server for carrying out the method according to claim 14 or 15.

19. Computer data signal embodied in a carrier wave and representing a program that instructs a computer to perform the steps of the method according to claim 14 or 15.
